# EUROPEAN PATENT SPECIFICATION

(11) **EP 3 585 261 B1**
(45) Date of publication and mention of the grant of the patent: **21.08.2024**
(21) Application number: 18756873.8
(22) Date of filing: 23.02.2018
(51) Int. Cl.: A61B 5/00

(54) **SYSTEM FOR DIAGNOSING SLEEP DISORDERS**
SYSTEME ZUR DIAGNOSE VON SCHLAFSTÖRUNGEN
SYSTÈME DE DIAGNOSTIC DE TROUBLES DU SOMMEIL

(30) Priority: 23.02.2017 US 201762462864 P
(43) Date of publication of application: 01.01.2020
(73) Proprietor: Indiana University Research & Technology Corporation, Indianapolis, IN 46202 (US)
(72) Inventor: CHEN, Peng-Sheng, Indianapolis IN 46278 (US); CHEN, Lan S., Indianapolis IN 46228 (US)
(74) Representative: V.O.
(86) International application number: PCT/US2018/019539
(87) International publication number: WO 2018/156967

(56) References cited:
- EP-A1- 2 286 723
- WO-A1-2014/089549
- US-A- 5 902 250
- US-A1- 2003 055 348
- US-A1- 2007 173 728
- US-A1- 2014 163 343
- US-A1- 2015 297 104

## Description

### BACKGROUND

The present disclosure relates generally to systems and methods for diagnosing and treating sleep disorders.

Obstructive sleep apnea is a common sleep disorder that affects millions of people. The disease is characterized by episodes involving obstruction of respiration at night, resulting in intermittent hypoxia. In addition, many patients suffer from sleep apnea without knowing it. If left untreated, sleep apnea can present a number of health problems, including fatigue, impaired concentration, depression, high blood pressure, stroke, heart failure and other conditions. Diagnosis of sleep apnea, and other sleep disorders, is readily achieved using sleep studies. Typically, sleep studies analyze air flow, body movements, electroencephalograms, electrocardiograms, and other physiological parameter to diagnose patients. However, many of these measurements often require cumbersome, complex and expensive technologies.

Therefore, there is a need for simple, low cost and user friendly approaches for efficiently screening for the presence of sleep disorders, such as sleep apnea. EP2286723 describes a method of diagnosing sleep apnea by extracting a series of RRI from an electrocardiogram (ECG) recorded on the chest of a sleeping subject.

### SUMMARY

The present disclosure overcomes the drawbacks of previous technologies by providing a system for use in diagnosing sleeps disorders. Specifically, a novel, noninvasive approach is introduced that relies on measurements of skin nerve activity to identify sleep disorders.

The present invention refers to a system for diagnosing a sleep disorder in a subject. The system includes a plurality of skin electrodes configured to sense electrical signals corresponding to a skin nerve activity in the subject. The system also includes a signal detector configured to sample the electrical signals sensed by the plurality of skin electrodes, and generate signal data using the electrical signals. The system further includes a processor configured to receive the signal data from the signal detector, and assemble a time-series of data indicating the skin nerve activity using the signal data. The processor is also configured to identify a sleep disorder using the time-series of data, and generate a report indicative of the sleep disorder.

Further, a method for diagnosing a sleep disorder in a subject is disclosed. The method includes sensing electrical signals corresponding to a skin nerve activity in the subject using a plurality of skin electrodes, and generating, using signal detector, signal data using the electrical signals. The method also includes assembling a time-series of data indicating the skin nerve activity using the signal data, and identifying a sleep disorder using the time-series of data. The method further includes generating a report indicative of the sleep disorder.

The foregoing and other advantages of the invention will appear from the following description.

### BRIEF DESCRIPTION OF THE DRAWINGS

FIG. 1 a schematic diagram of an example system for use in diagnosing a sleep disorder, in accordance with aspects of the present disclosure.
FIG. 2A is an example of the system shown in FIG. 1.
FIG. 2B is an illustration showing one non-limiting configuration of the system shown in FIG. 1.
FIG. 2C is another illustration showing another non-limiting configuration of the system shown in FIG. 1.
FIG. 3A are graphs showing a differential filter being applied to an artificially generated electrical signal.
FIG. 3B are graphs showing different high pass settings selective for skin nerve activity, in accordance, with aspects of the present disclosure.
FIG. 4 is a flowchart setting forth steps of a process, in accordance with aspects of the present disclosure.
FIG. 5 are graphs illustrating correlations between skin nerve activity and episodes of obstructive sleep apnea, in accordance with aspects of the present disclosure.
FIG. 6 are graphs illustrating measurements of skin nerve activity, electrocardiogram and heart from a patient exhibiting atrial tachycardia (AT) with aberrant ventricular conduction.
FIG. 7 are graphs illustrating sleep study measurements indicating obstructive sleep apnea with sympathetic activation.
FIG. 8 is a graph comparing average skin nerve activity of patients with and without sleep disturbed breathing.

### DETAILED DESCRIPTION

The present invention refers to a system according to the claims for diagnosing a sleep disorder in a subject. The present disclosure introduces a novel approach for diagnosing sleep disorders, such as sleep disturbed breathing (SDB) disorders, in a subject. In particular, obstructive sleep apnea (OSA) is a SDB disorder in which the upper airway becomes blocked repeatedly during sleep, often reducing or completely stopping air flow. This presents a significant problem from a public health perspective because patients diagnosed with sleep apnea have higher risks for asthma, cardiovascular disease, chronic kidney disease, cognitive and behavioral disorders, and otherdiseases.

Multiple mechanisms have been suggested to explain how OSA can increase the risk for cardiovascular and other diseases. One of the mechanisms relates to sympathetic nerve activation. Therefore, several studies have been performed to investigate changes in sympathetic neural traffic during sleep. In particular, measurements of muscle (MSNA) sympathetic nerve activity have been performed and revealed appreciable changes during sleep and sleep-related episodes of OSA. In addition, in one study, investigators utilized microneurography techniques to record muscle and skin sympathetic nerve traffic to assess whether the adrenergic overdrive seen in muscle circulation is detected in cutaneous circulation. However, results of that study indicated a lack of sympathetic skin nerve activation with OSA. In addition, microneurography measurements require invasive techniques that prevent widespread adoption for diagnosing OSA. Furthermore, microneurography cannot be used in ambulatory patients over prolonged periods of time.

By contrast, it is a discovery of the present disclosure that non-invasive measurements of skin nerve activity may be used to diagnose sleep disorders. Specifically, electrical signals corresponding to skin nerve activity may be acquired using skin electrodes and used identify a sleep disorder, like OSA. In addition, such approach significantly reduces potential risks and complications associated with more invasive procedures, and advances clinical translation.

The description below and the accompany figures provide a general understanding of the environment for the system disclosed herein as well as the details for the system. In the drawings, like reference numerals are used throughout to designate like elements.

As used herein, the term "electrode" refers to any sensor or element that includes an electrical conductor configured to sense electrical signals from a subject, such as electrical signals from biological tissue, nerve activity and so on, when coupled to or brought into contact with the subject or subject's skin.

As used herein, the term "cutaneous" as applied to use of electrodes or skin electrodes refers to placing electrodes on the surface of the skin of a subject without puncturing the skin of the subject. As described below, the cutaneous electrodes detect electrical activity associated with nerves that are proximate to the skin of the subject, including sympathetic nerves in the autonomic nervous system that innervate the skin.

As used herein, the term "subcutaneous" as applied to use of electrodes or skin electrodes refers to placing electrodes entirely underneath the skin with leads from the electrodes being electrically connected to a device that is placed in the body of the test subject, such as an internal pacemaker, defibrillator, or cardiac resynchronization device. The subcutaneous electrodes described herein are different than electrodes that are used in prior art microneurography procedures. First, the subcutaneous electrodes are completely under the skin, with no portion of the electrode or lead extending through the skin. Second, the subcutaneous electrodes do not have to be placed in close proximity to a particular nerve fiber to be used in detection of electrical signals from nerve activity. Third, the subcutaneous electrodes are shaped with a blunt contact surface without the sharp needle tips of microneurographic electrodes, which enables the subcutaneous electrodes to remain under the skin of an ambulatory subject for long term monitoring of nerve activity without injuring the subject. Fourth, the metal housing of an implanted device can be used to house subcutaneous electrodes in some embodiments. In the latter situation, no additional electrodes are needed.

In both the cutaneous and subcutaneous configurations described above, the electrodes are located proximate to nerves that innervate the skin. As is known in the medical art, many nerves that innervate the skin are part of the sympathetic nervous system, which is in turn part of the autonomic nervous system in humans and many animals. Different nerve fibers in the sympathetic nervous system also innervate cardiac tissue, as well as other muscles and organs in the body. For example, the sympathetic nervous system is associated with the "fight or flight" response where the sympathetic nervous system activity increases and the pupils dilate, the heart rate increases, bronchioles in the lungs dilate, blood vessels near the surface of the skin constrict, and the sweat glands secrete sweat at a higher rate. The sympathetic nervous system is also associated with the "sympathetic outflow" process that occurs when a subject awakens from sleep. While the sympathetic nervous system includes a large number of nerve bundles that innervate different parts of the body in a subject, the nerves in the sympathetic nervous system are associated with each other and the level of activity in one nerve fiber often corresponds to the level of activity in other nerve fibers in the sympathetic nervous system.

As used herein, terms such as "approximately," "around," "about," and other synonyms, in relation to stated numerical values may include variations as understood by one of ordinary skill in the art. In some aspects, such variation may be negative (less than a nominal value) or positive (more than a nominal value), and up to 10%, or more, of the stated nominal value(s).

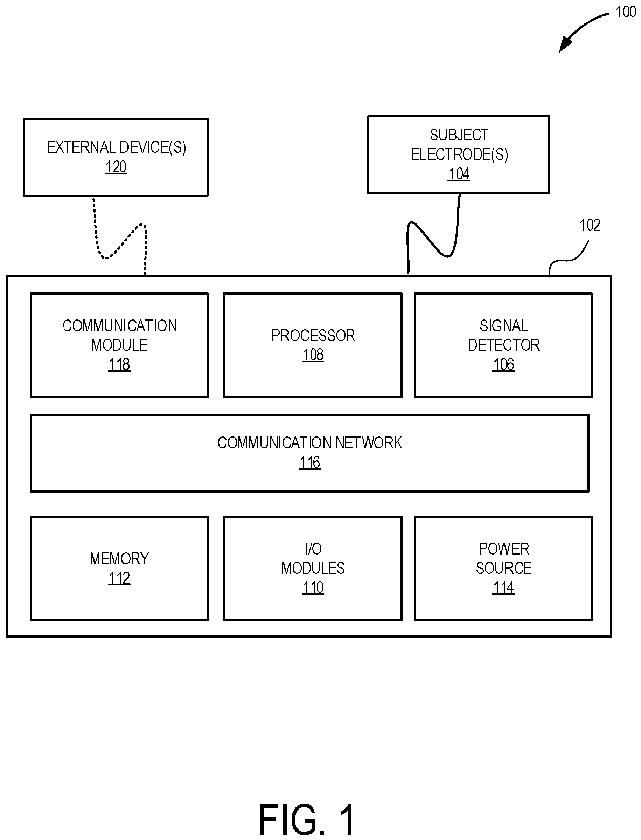

Turning to FIG. 1, a non-limiting example of a system 100 for use in diagnosing sleep disorders, in accordance with aspects of the present disclosure, is shown. The system 100 includes a monitor unit 102 and a number of subject electrodes 104 connectable thereto. In general, the monitor unit 102 includes a signal detector 106, a processor 108, various input/output ("I/0") modules 110, a memory 112, and a power source 114. The monitor unit 102 also includes a communication network 116 configured to facilitate the transfer of data, signals and other information between the various elements of the monitor unit 102. As shown, the monitor unit 102 may also include a communication module 118 that allows data and information exchange between the monitor unit 102 and various external device(s) 120, such computers, databases, servers, internet, cloud, and so on. Above-mentioned elements of the monitor unit 102, or various components thereof, may be housed in one or more housing units or enclosures.

In some embodiments, the electrodes 104 may include a number of skin electrodes. For example, in one non-limiting configuration, the skin electrodes include a reference electrode and two input electrodes. In particular, the skin electrodes are configured to sense electrical signals corresponding to skin nerve activity in the subject. To do so, the skin electrodes may be designed to engage the subject's skin cutaneously and/or subcutaneously. In addition, the skin electrodes may be constructed using various materials (e.g. conductors, semi- conductors, insulators, metals, elastics, plastics, and others), and have various dimensions. The skin electrodes may also be reusable or disposable.

Electrical contact of the skin electrodes with the subject's skin may be achieved using dry contact or wet contact, as well as other techniques known in the art. Also, electrical contact may be maintained using adhesives, bandages, straps, belts, suction cups, clips and other methods. In some implementations, the skin electrodes may be patch electrodes or standard electrocardiogram ("ECG") electrodes. In other configurations, the skin electrodes may be integrated in various objects, garments, and thelike.

Optionally, the system 100 may also include other sensors that may be coupled to the monitor unit 102 (not shown). These sensors may be configured to measure various other physiological signals from the subject, including signals corresponding to heart rate, respiration, oxygenation, and so forth.

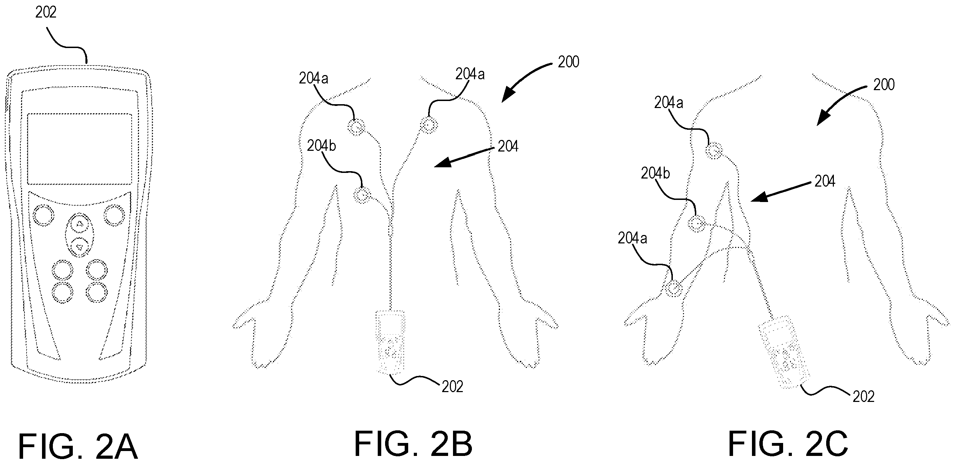

In some implementations, the system 100 may be a stand-alone system, a wearable or portable device or apparatus. For instance, FIGs. 2A-2C illustrate one non-limiting example of a portable device 200. In particular, the portable device 200 may include a monitor unit 202 and a number of patch electrodes 204. The monitor unit 202 may include various features, including capabilities for receiving electrical signals detected by the patch electrodes 204 and analyzing the signals to identify a sleep disorder. In some implementations, the monitor unit 202 may be configured to acquire, preprocess, store and report data corresponding to the electrical signals, as described. In one non-limiting example, the portable device 200 may be similar to, or have similar capabilities as, the ME6000 Biomonitor (Biomation, Almonte, Ontario).

In some configurations, the skin electrodes 204 of the portable device 200 may include input electrodes 204a for detecting skin nerve activity or cardiac activity, and reference electrodes 204b to be used as a reference. Of course, the portable device 200 may include fewer or more skin electrodes 204, which may be arranged in any manner depending on desired measurements from the subject. For instance, in one configuration, one channel of the portable device 200 may be used to record ECG Lead I (FIG. 2B), while another channel may be used to record skin nerve activity (FIG. 2C).

Referring again to FIG. 1, the signal detector 106 is configured to sample the electrical signals sensed by the electrodes 104, and generate signal data using the electrical signals. The signal detector 106 may then direct the signal data to the processor 108 for further processing and analysis. In some implementations, the signal detector 106 may include one or more amplifiers (e.g. differential amplifiers) electrically coupled to electrodes 104. The amplifiers may be configured to amplify voltage signals, or differential voltage signals, for example, sensed by the electrodes 104. The signal detector 106 may also include other elements or circuitry for pre-processing raw signals sensed by the electrodes 104. For instance, the signal detector 106 may include various input filters (e.g. low-pass, bandpass, high-pass). According to the invention a bandpass input filter extends over a frequency range between approximately 500 Hz and approximately 1000 Hz. In another non-limiting example, a bandpass input filter extends over a frequency range between approximately 0.5 Hz and approximately 150 Hz.

The signal detector 106 may also include one or more samplers configured to sample signals (e.g. raw, filtered, and/or amplified signals) at a predetermined sampling rate and generate the signal data. In some implementations, samplers may include various analog-to- digital converters ("ADCs"), as well as other data acquisition hardware or sampling circuitry. For instance, samplers may be configured to generate signal data at a sampling rate between approximately 4,000 and approximately 10,000 samples per second, although other sampling rates may also be possible. As non-limiting examples, the signal detector 106 may be similar to, or have similar capabilities as, the ML138 or ML135 OctoBioAmp (ADInstruments, Colorado Springs, CO).

The processor 108 may include one or more microcontrollers, microprocessors, or other processing units, that are configured or programmed to perform various steps for operating the system 100. In some aspects, the processor 108 may execute steps based on user input providing operational instructions or selections, for example. However, user input is not required, and the processor 108 may be configured to operate autonomously. In addition to operating the system 100, the processor 108 may also be configured to execute steps for identifying a sleep condition of a subject, in accordance aspects of present disclosure. To do so, the processor 108, or processing unit therein, may execute non-transitory programming, or instructions hardwired therein. In this regard, the processor 108 would therefore be application- specific.

Alternatively, or additionally, the processor 108 may be, or include, a general- purpose processor configured to access and execute instructions stored the memory 110. For example, the processor 108 may include a central processing unit ("CPU") with one or more cores, and optionally a graphical processing unit ("GPU"). The processor 108 may also include various digital logic devices, including application specific integrated circuits ("ASICs"), field programmable gate arrays ("FPGAs"), and digital signal processor ("DSP") devices. In embodiments of the system 100, the processor 108 may also include low-power digital logic devices that enable long-term operation of the system 100.

As described in more detail below, the electrical activity in the nerves that innervate the skin occurs at higher frequencies and lower amplitudes compared to the electrical signals generated in the cardiac muscle during a heartbeat. As such, processor 102 may be configured to select signal data corresponding to specific signals in the subject, such as skin nerve or cardiac activity, by processing signal data received from the signal detector 108. That is, the processor 102 may apply appropriate filters, such as low-pass filters, high-pass filters, or bandpass filters, to the data to obtain signals of interest. The processor 102 may also scale, multiply, integrate or average signal data over a predetermined period of time. In some aspects, the processor 102 may select the predetermined period of time for integrating or averaging the signal data that is suitable to achieve a desired signal-to-noise ratio **(SNR).** For example, the integration time or average time may be between 10 ms and 500 ms.

For example, a high-pass filter (e.g. 3 dB) with a cutoff frequency adjustable in a range of between approximately 100 Hz and approximately 1 kHz may be utilized by the processor 108. Selection of the proper high-pass setting might require consideration of signal specificity and acceptable sensitivity. For instance, a filter with a high-pass cutoff frequency of approximately 150 Hz would be sufficient to attenuate most the lower frequency signals from cardiac muscle activity and electrical signals from other muscles in the subject typically observed, but not all muscle noise. On the other hand, a cutoff at approximately 700 Hz would be more specific to nerve activity, as the muscle noise does not generate signals with frequencies above approximately 500 Hz, but such filter setting would result in a reduced measurement sensitivity. Therefore, in some implementations, the high-pass filter cutoff frequency may be between approximately 150 Hz and approximately 700 Hz, although other values may be possible. In other implementations, a bandpass filter selective for skin nerve activity may be applied, where the filter extends over a frequency range between approximately 500 Hz and approximately 1000 Hz.

In some aspects, ECG monitoring may be desired. As such, signal data from the signal detector 104 may be processed using a low-pass filter, for example, with a cutoff frequency approximately in a range between 0.5 Hz and 150 Hz in order to detect cardiac activity. Alternatively, a bandpass filter may be applied. For example, the bandpass filter may have a lower cutoff frequency of approximately 0.5 Hz and an upper cutoff frequency of approximately 150 Hz. In some implementations, the same pair of electrodes 104 (e.g. patch electrodes) may be used to simultaneously record ECG and skin nerve activity from the surface of thoracic skin, for example, or elsewhere. In such case, the signal data may be used to determine both ECG and skin nerve activity. As such, the signal data may be low-pass filtered (selective ECG signals) and high-pass filtered (selective for skin nerve activity). Additionally, where an alternating current ("AC") electrical signal is used to supply power to one or more components in system 100, a bandpass filter (e.g. a notch-filter) that attenuates frequencies near the primary frequency of the AC signal, such as 50 Hz or 60 Hz may also be applied.

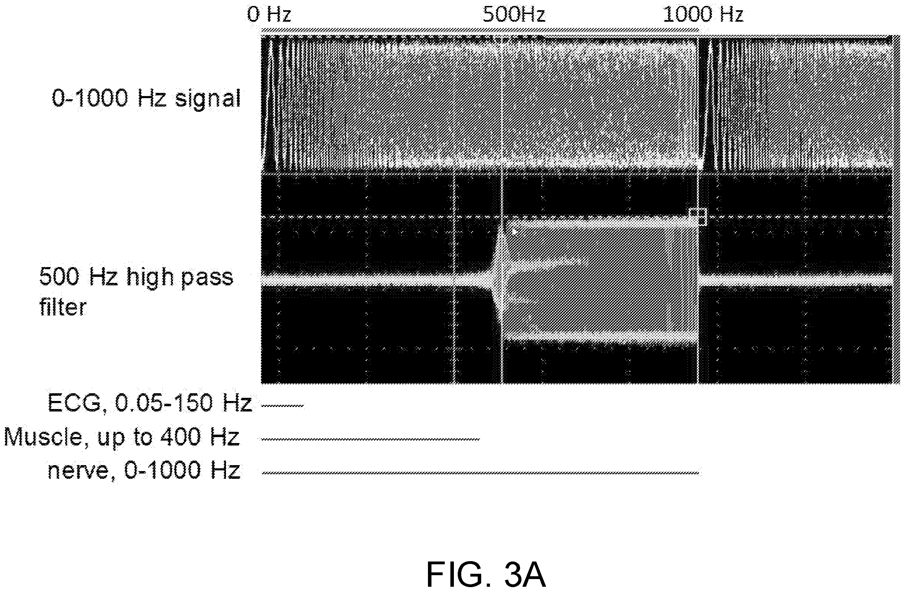

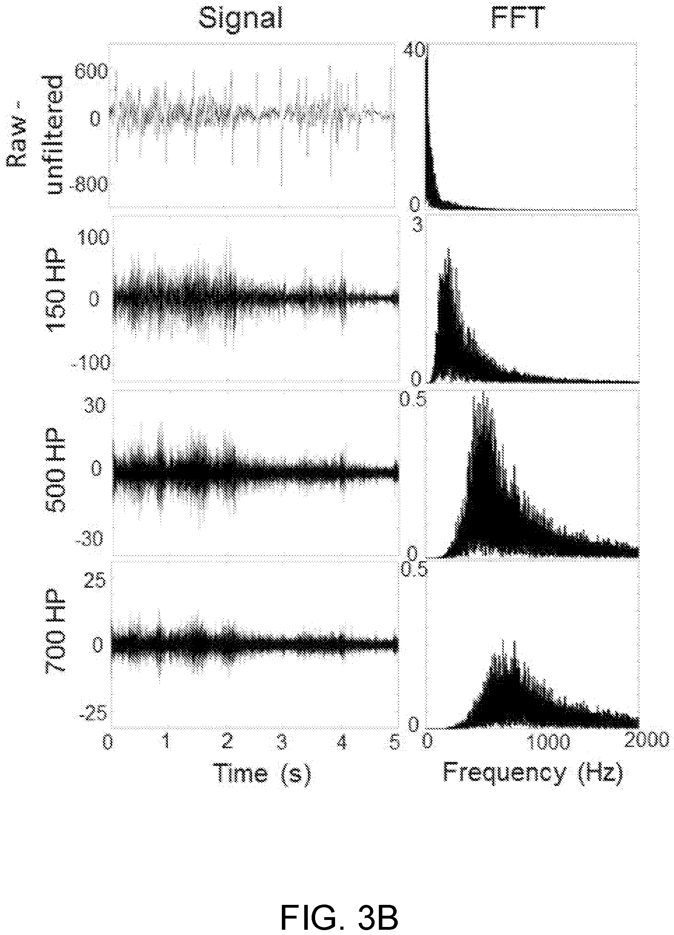

By way of example, FIG. 3A shows a differential filter of an artificially generated electrical signal with frequencies varied from Oto 1000 Hz (top graph). High-pass filtering with a cutoff frequency of 500 Hz or bandpass filtering selecting frequencies between 500 Hz and 1000 Hz eliminated the lower frequency signals (bottom graph). In another example, FIG. 3B illustrates the concept of applying various high-pass filter to raw signals containing both ECG and skin nerve activity. Applying high-pass (HP) filters with cutoff frequencies above approximately 150 Hz eliminated the ECG portion of the signal. As illustrated by the Fast Fourier Transform (FFT) of the filtered signals, higher filter setting result in a reduced measurement sensitivity.

Referring again to FIG. 1, in accordance with aspects of the present disclosure, the processor 108 may be configured to receive the signal data generated by the signal detector 104, and use the signal data to assemble a time-series of data indicating or selective for skin nerve activity, as described. The processor 108 may then identify a sleep disorder, such as a SDB, by performing various analyses of the time-series of data. In one example, the processor 108 may compare a portion of the time-series of data to a baseline or a predetermined skin nerve activity signature. In another example, the processor 108 may compute an average value of the skin nerve activity and compare the average to a predetermined value or threshold. In yet another example, the processor 108 may determine whether an average value of the skin nerve activity exceeds a baseline value by more than a value between approximately 0.1 microvolts and approximately 0.5 microvolts. In addition, identifying the sleep disorder, the processor 108 may also take into consideration other information about the subject, including age, gender, medical condition, and so on, as well as other data obtained from the subject, such as various physiological measurements.

The processor 108 may then generate a report. The report may be in any form, and include any information. For instance, the report may include various waveforms, indicating various measured parameters, including skin nerve activity (e.g. average skin nerve activity), air flow, oxygenation, heart rate, episodes of **OSA,** and so forth. The report may be directed to a display, stored in a memory, or transmitted electronically. In some aspects, the report may be transmitted as a message, page, email, text message, or other report form, to alert a remote healthcare professional of the identified event.

The I/O modules 110 of the monitoring system 100 may be configured to receive a wide variety of data, information, as well as selections or operational instructions from a user. To this end, the I/0 modules 110 may include various elements for receiving input, including buttons, switches, toggles, knobs, touch screens, or other touch-responsive elements, as well as ports, connectors, and receptacles for flash-memory, USB sticks, cables, and so on. The I/O modules 110 may also be configured to provide a report by way of various output elements, including screens, displays, LEDs, LCDs, speakers and so on.

The memory 112 may include various memory elements where a number of types of data (e.g., internal data, external data instructions, software codes, status data, diagnostic data, etc.) may be stored. As an example, the memory 112 may include random access memory ("RAM"), dynamic random access memory ("DRAM"), electrically erasable programmable read- only memory ("EEPROM"), flash memory, and the like. In some implementations, the memory 112 may also include non-transitory computer-readable media, which may include instructions executable by the processor 108 for operating the system 100 as well as carrying out methods in accordance with present disclosure. The memory 112 may also store a variety of other data and information, including reference or baseline data representing skin nerve activity signatures and corresponding sleep disorders, such as OSA. Such data may be stored as waveforms, time- series, tables and other data representations.

The power source 114 is configured to power various elements and circuitry in the monitor unit 102. In one example, the power source 114 may include a rechargeable or replaceable battery. In another example, the power source 114 may be configured to receive power from an external source of power, such as an AC outlet.

The communication network 116 may include a variety of communication capabilities and circuitry, including various wiring, components and hardware for electronic, radiofrequency ("RF"), optical and other communication methods. By way of example, the communication network 116 may include parallel buses, serial buses, and combinations thereof. Example serial buses may include serial peripheral interface (SPI), I2C, DC-BUS, UNI/0, 1- Wire, and others. Example parallel buses may include ISA, ATA, SCSI, PIC, IEEE and others.

The communication module 118 may be configured to facilitate communications between the system 100 and the external device(s) 120. To this end, the communication module 118 may include any hardware, software, and firmware capable of achieving wired or wireless communication. In some implementations, the communication module 118 may be configured perform wireless communication using RF, WiFi, Bluetooth or other wireless communication protocols.

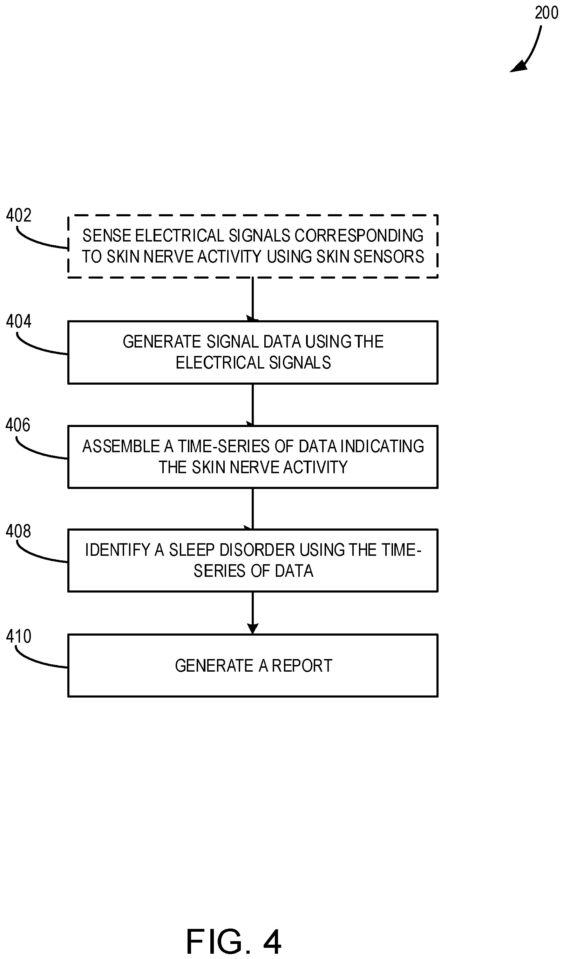

Referring now to FIG. 4, steps of a process 400, in accordance with aspects of the present disclosure, are shown. The process 300 may be carried using a system 100, as described with reference to FIGs. 1-2, or another suitable system, device or apparatus. Steps of the process 400 may be implemented as a program, firmware, or executable instructions hardwired or stored in non-transitory computer readable media.

The process 400 may optionally begin at process block 302 with sensing electrical signals corresponding to skin nerve activity. As described, electrical signals may be cutaneous and/or subcutaneous electrodes coupled to a subject (e.g. skin electrodes). In some configurations, three or more electrodes, may be placed on the subject in a cutaneous configuration. Electrodes may also be implanted under the skin of the subject in a subcutaneous configuration, although other arrangements are possible. In some implementations, electrical signals may be preprocessed, for example by being amplified to generate amplified signals. Optionally, the raw or amplified signals may also be filtered to generate filtered signals, as described.

Then, at process block 404, signal data may be generated using the electrical signals. As described, this step may include sampling the sensed electrical signals at a predetermined sampling rate using a sampler. In some aspects, the generated signal data may be alternatively retrieved from a memory at process block 404. The signal data may then be used to assemble a time-series of data indicating skin nerve activity, as indicated by process block 406.

In assembling the time-series of data at process block 406, one or more filters (e.g. low-pass, high-pass, bandpass) may be applied to the signal data. In some aspects, the filter may be selective of skin nerve activity. To this end, the filter may be configured to attenuate frequencies in the signal data corresponding to muscle activity, and other sources of interference. For example, a bandpass filter that extends over a frequency range between approximately 500 Hz and approximately 1000 Hz. As described, other signals may also be desirable, including ECG signals. To this end, the same or different signal (i.e. obtain using different electrodes) data may be used to provide those signals. In some aspects, electrocardiogram (ECG) data may be generated by applying a bandpass filter to the signal data, wherein the bandpass filter extends over a frequency range between approximately 0.5 Hz and approximately 150 Hz. Other signal filtering, as well as processing steps may also be possible at process block 406, including scaling, multiplying, or integrating signal data.

Then, at process block 408, the assembled time-series of data may be analyzed to identify a sleep disorder. As described, this step may include comparing a portion of the time-series of data indicating skin nerve activity to a baseline. Also, this step may include comparing an average value of the skin nerve activity to a predetermined threshold.

A report may then be generated at process block 410. The report may be provided in substantially real time, for example, using a display, or stored in a memory to be retrieved at a later time. In some aspects, the report may be in the form of graphs or time traces of monitored skin nerve activity. Displayed or retrieved activities corresponding to monitored or estimated nerve activities may then utilized by a doctor or other healthcare professional during or following the course of medical treatment for a subject. The report may also include information derived from measurements skin nerve activity, including average signals, signal variations, signal frequencies, frequency variations, identified events, event timings, deviations from a baseline, identified sleep disorders, and so forth.

In some implementations, above-described steps may be carried out in a passive operating mode, displaying the skin nerve activity and recording activity in the memory for subsequent retrieval and analysis. Subsequently, a doctor or other healthcare provider would retrieve and review information or data associated with the time-series of data as part of diagnosis and treatment in a subject. The passive operating mode can be used, for example, during diagnosis of a medical condition, during long-term monitoring of a subject to assess progress in a course of medical treatment, and for studies of subjects during clinical trials or other scientific research.

In other implementations, above steps may be carried out to generate a baseline measurement of skin nerve activity in a subject. For example, the baseline nerve activity can include an average signal amplitude, or signal variation with time. The baseline could then be used to determine a change in the level of skin nerve activity over time, for example, as a result of a change in medical condition, such as an OSA event. A determined rapid change deviating from the baseline by more than a predetermined value or threshold, could then initiate an audio or visual alarm to a clinician in response to the identified change in nerve activity.

The above-described system may be further understood by way of examples. These examples are offered for illustrative purposes only, and are not intended to limit the scope of the present invention in any way. Indeed, various modifications of the invention in addition to those shown and described herein will become apparent to those skilled in the art from the foregoing description and the following examples and fall within the scope of the appended claims. For example, certain electrode arrangements and configurations are presented, although it may be understood that other configurations may be possible, and still considered to be well within the scope of the present invention. Likewise, specific process parameters and methods are recited that may be altered or varied based on variables such as signal amplitude, phase, frequency, duration, and so forth.

### EXAMPLE

Sleep-disordered breathing (SDB) refers to a wide spectrum of sleep-related breathing abnormalities, including obstructive sleep apnea-hypopnea syndrome (OSA). Cardiac arrhythmias are frequently observed during sleep studies. In one study, the authors reported that 193 (48%) of 400 patients had cardiac arrhythmias during the recorded night. In particular, there is an association between OSA and atrial fribrillation/atrial tachycardia, and that successful treatment of OSA reduces arrhythmia recurrences after cardioversion and catheter ablation. Previous studies showed that muscle sympathetic nerve activity (MSNA) as well as circulating plasma norepinephrine are increased in awake patients with OSA. Successful Continuous positive airway pressure (CPAP) therapy decreased MSNA from 69.4±15.3 bursts/min to 53.9±10.5 bursts/min, a 22% reduction. Another study found that CPAP therapy reduced mean 24-hr HR from 83.0 ±11.5 to 79.7±9.8 (P < 0.002) in the CPAP group compared with a non- significant rise in the subtherapeutic control group. These findings suggest that patients with OSA have increased sympathetic tone, which was reduced by CPAP therapy.

Successful CPAP therapy of OSA is associated with reduced AF recurrences after cardioversion and catheter ablation. However, due to difficulties in obtaining stable impalements, long term continuous microneurography recording in large number of patients is difficult. What percentage of patients with OSA has elevated sympathetic tone remains unclear. It is possible that the effects of OSA on individual patients are determined primarily by a reduction of sympathetic tone. Therefore, a biomarker is needed to select patients with large elevation of sympathetic tone for more intensive therapy to prevent cardiovascular events, such as cardiac arrhythmias. OSA is therefore an excellent human model of spontaneous atrial tachyarrhythmia associated with sympathetic activation.

It has been proposed that future investigations clarifying the contribution of specific OSA-related mechanistic pathways to arrhythmia generation may allow targeted preventative therapies to mitigate OSA-induced arrhythmogenicity. Furthermore, interventional studies are needed to clarify the impact of OSA pathophysiology reversal on cardiac arrhythmogenesis and related adverse outcomes.

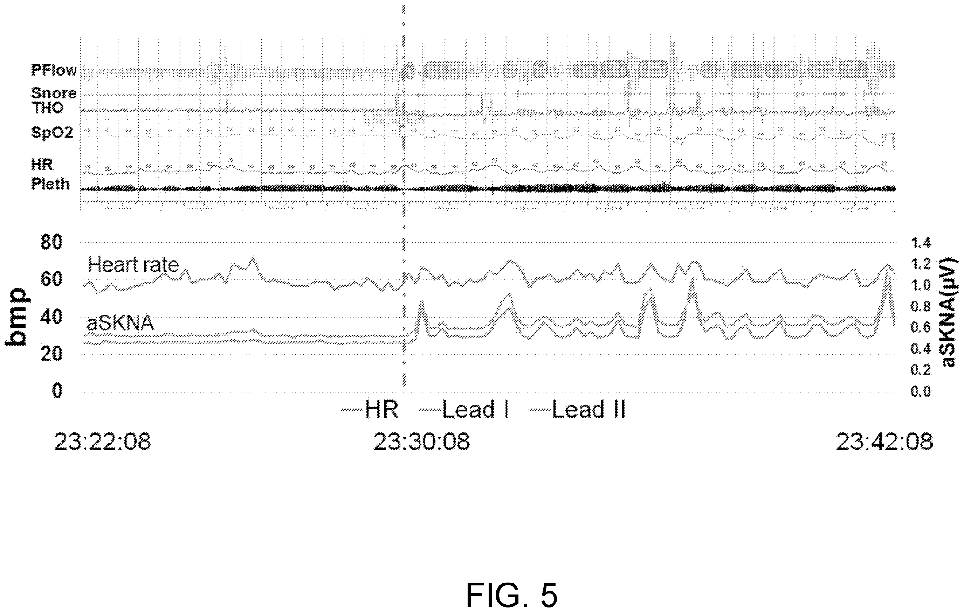

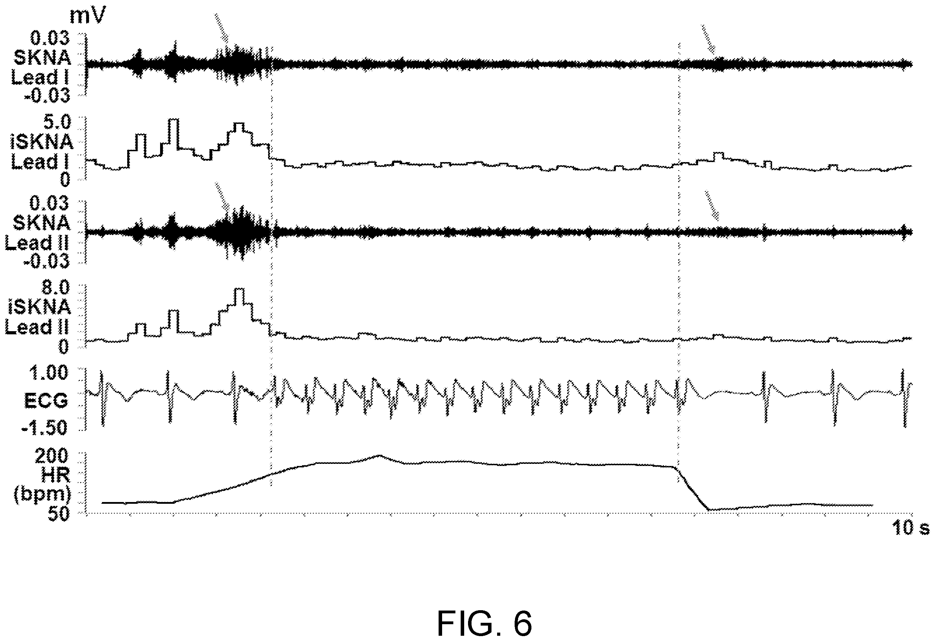

A preliminary study on a 62-year-old man with known atrial tachyarrhythmias referred for sleep study was performed. A ME6000 neuECG recorder was placed on the chest with electrodes connected to the chest and right arm as illustrated in FIG. 2A. The patient was also connected to a home sleep study unit. Sleep study data obtained from the patient is shown in FIG. 5. Specifically, the study shows the onset of intermittent nasal flow obstruction and hypoxia at around 23:30:08 (red vertical dotted line). Measurements shown in FIG. 5 include the heart rate (HR) in beats per min (bpm) pressure from nasal pressure transducer (Pflow), activity from thoracic channel (THO), peripheral capillary oxygen saturation (Sp02), pulse rate (PR), and plethysmograph measurements (Pleth). The annotations (rectangles) in top panel of FIG. 5 indicate obstructive apneas and hypopneas. neuECG recordings of AT with aberrant ventricular conduction were also obtained (FIG. 6).

Referring particularly to FIG. 5, the patient exhibited OSA starting at about 11:30pm with intermittent hypoxic episodes. Simultaneous recording of skin nerve activity (SKNA) shows a coincidental increase at 11:30 pm, which corresponded to the onset of OSA. Note that the onset of OSA was associated with simultaneous elevation of SKNA in both Lead I and Lead II. Intermittent SKNA activity was associated with increased HR. These findings indicate that the development of OSA was associated with elevated sympathetic tone in this patient. The neuECG recordings showed multiple episodes of AT in this patient, as shown in FIG. 6. Arrows point to elevated SKNA 10-s before and after termination of AT. Vertical dotted lines indicate onset and termination of AT. After CPAP therapy, the patient no longer had palpitations and there were no recurrences of arrhythmia at I-year follow up. The patient did not take antiarrhythmic medications.

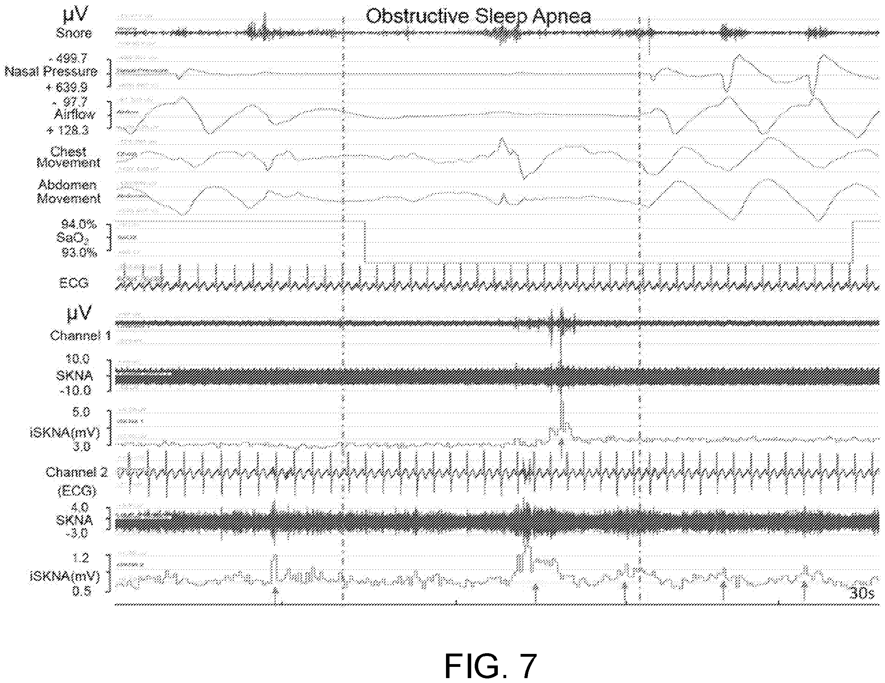

In addition to patient studied above, a total of 11 patients were monitored in a sleep study. Among them, 8 had a diagnostic study of SDB and the other 3 had a normal sleep study. Data from a representative patient with OSA is shown in FIG. 7. As noted in the figure, an elevation in the average skin nerve activity (aSKNA) elevation was observed. Specifically, robust SKNA discharges were present (red arrows). The AHiI was 19.8. The integrated skin nerve activity (iSKNA) was integrated over every 100 ms.

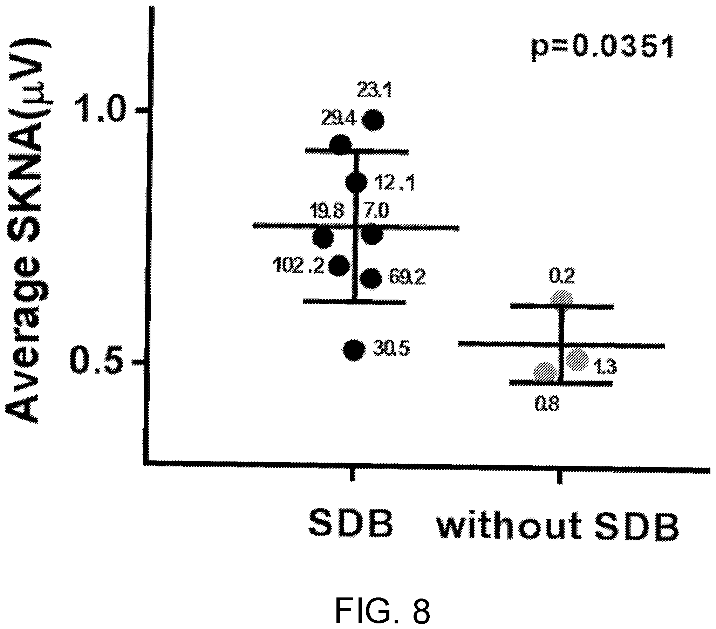

FIG. 8 shows the apnea-hypopnea index (AHI) and aSKNA in all patients. Note that aSKNA in patients with SDB is higher than those without SDB. These preliminary data suggest that the effect size of the aSKNA between those with and without SDB is about 1.5. Also, AHI and aSKNA are poorly correlated, indicating that AHI is a poor indicator of elevated sympathetic tone.

The various configurations presented above are merely examples and are in no way meant to limit the scope of this disclosure. Variations of the configurations described herein will be apparent to persons of ordinary skill in the art.

In particular, features from one or more of the above-described configurations may be selected to create alternative configurations comprised of a sub- combination of features that may not be explicitly described above. In addition, features from one or more of the above-described configurations may be selected and combined to create alternative configurations comprised of a combination of features which may not be explicitly described above. Features suitable for such combinations and sub-combinations would be readily apparent to persons skilled in the art upon review of the present application as a whole.

## Claims

1. A system (100, 200) for diagnosing a sleep disorder in a subject, the system comprising:
a plurality of skin electrodes (104, 204) configured to sense electrical signals corresponding to a skin nerve activity in the subject;
a signal detector (106) configured to sample the electrical signals sensed by the plurality of skin electrodes (104, 204), and generate signal data using the electrical signals;
a processor (108) configured to:
receive the signal data from the signal detector (106);
assemble a time-series of data indicating the skin nerve activity using the signal data, including applying a bandpass filter that extends over a frequency range between approximately 500 Hz and approximately 1000 Hz to the signal data;
identify a sleep disorder using the time-series of data; and
generate a report indicative of the sleep disorder.

2. The system of claim 1, wherein the skin electrodes (104, 204) comprises patch electrodes (104, 204) configured to couple to a surface of the subject's skin.

3. The system of claim 1, wherein the signal detector (106) comprises signal amplifiers that are electrically connected to the plurality of skin electrodes (104, 204) and configured to generate amplified electrical signals.

4. The system of claim 3, wherein the signal detector (106) is configured to generate the signal data using the amplified electrical signals.

5. The system of claim 1, wherein the processor (108) is further configured to generate electrocardiogram (ECG) data by applying a bandpass filter to the signal data, wherein the bandpass filter extends over a frequency range between approximately 0.5 Hz and approximately 150 Hz.

6. The system of claim 1, wherein the processor (108) is further configured to identify the sleep disorder by comparing the skin nerve activity to a baseline, or wherein the processor (108) is further configured to identify a sleep disturbed breathing (SDB) using the time-series of data.

## Patentansprüche

1. System (100, 200) zur Diagnose einer Schlafstörung bei einer Person, wobei das System umfasst:
eine Vielzahl von Hautelektroden (104, 204), konfiguriert, um elektrische Signale zu erfassen, die einer Hautnervenaktivität bei der Person entsprechen;
einen Signaldetektor (106), konfiguriert, um die von der Vielzahl von Hautelektroden (104, 204) erfassten elektrischen Signale abzutasten und Signaldaten unter Verwendung der elektrischen Signale zu erzeugen;
einen Prozessor (108), konfiguriert zum:
Empfangen der Signaldaten von dem Signaldetektor (106);
Erstellen einer Zeitreihe von Daten zum Anzeigen der Hautnervenaktivität unter Verwendung der Signaldaten, einschließlich des Anwendens eines Bandpassfilters, der sich über einen Frequenzbereich zwischen etwa 500 Hz und etwa 1000 Hz auf die Signaldaten erstreckt;
Identifizieren einer Schlafstörung unter Verwendung der Zeitreihe von Daten; und
Erzeugen eines Berichts über die Schlafstörung.

2. System nach Anspruch 1, wobei die Hautelektroden (104, 204) Pflasterelektroden (104, 204) umfassen, konfiguriert, um sich mit einer Oberfläche der Haut der Person zu verbinden.

3. System nach Anspruch 1, wobei der Signaldetektor (106) Signalverstärker umfasst, die mit der Vielzahl der Hautelektroden (104, 204) elektrisch verbunden sind und konfiguriert sind, um verstärkte elektrische Signale zu erzeugen.

4. System nach Anspruch 3, wobei der Signaldetektor (106) konfiguriert ist, um die Signaldaten unter Verwendung der verstärkten elektrischen Signale zu erzeugen.

5. System nach Anspruch 1, wobei der Prozessor (108) ferner konfiguriert ist, Elektrokardiogramm (EKG)-Daten zu erzeugen, durch Anwenden eines Bandpassfilters auf die Signaldaten, wobei sich der Bandpassfilter über einen Frequenzbereich zwischen etwa 0,5 Hz und etwa 150 Hz erstreckt.

6. System nach Anspruch 1, wobei der Prozessor (108) ferner konfiguriert ist, die Schlafstörung zu identifizieren, durch Vergleichen der Hautnervenaktivität mit einer Grundlinie, oder wobei der Prozessor (108) ferner konfiguriert ist, eine schlafgestörte Atmung (*Sleep Disturbed Breathing* - SDB) unter Verwendung der Zeitreihe von Daten zu identifizieren.

## Revendications

1. Système (100, 200) pour diagnostiquer un trouble du sommeil chez un sujet, le système comprenant :
une pluralité d'électrodes cutanées (104, 204) configurées pour détecter des signaux électriques correspondant à une activité nerveuse cutanée chez le sujet ;
un détecteur de signal (106) configuré pour échantillonner les signaux électriques détectés par la pluralité d'électrodes cutanées (104, 204) et générer des données de signal en utilisant les signaux électriques ;
un processeur (108) configuré pour :
recevoir les données de signal provenant du détecteur de signal (106) ;
assembler une série chronologique de données indiquant l'activité nerveuse cutanée en utilisant les données de signal, notamment en appliquant un filtre passe-bande qui s'étend sur une plage de fréquences comprise entre environ 500 Hz et environ 1 000 Hz aux données de signal ;
identifier un trouble du sommeil en utilisant la série chronologique de données ; et
générer un rapport indicatif du trouble du sommeil.

2. Système selon la revendication 1, dans lequel les électrodes cutanées (104, 204) comprennent des électrodes à plaque (104, 204) configurées pour se coupler à une surface de la peau du sujet.

3. Système selon la revendication 1, dans lequel le détecteur de signal (106) comprend des amplificateurs de signal qui sont électriquement connectés à la pluralité d'électrodes cutanées (104, 204) et configurés pour générer des signaux électriques amplifiés.

4. Système selon la revendication 3, dans lequel le détecteur de signal (106) est configuré pour générer les données de signal en utilisant les signaux électriques amplifiés.

5. Système selon la revendication 1, dans lequel le processeur (108) est en outre configuré pour générer des données d'électrocardiogramme (ECG) en appliquant un filtre passe-bande aux données de signal, dans lequel le filtre passe-bande s'étend sur une plage de fréquences comprise entre environ 0,5 Hz et environ 150 Hz.

6. Système selon la revendication 1, dans lequel le processeur (108) est en outre configuré pour identifier le trouble du sommeil en comparant l'activité nerveuse cutanée à une ligne de base, ou dans lequel le processeur (108) est en outre configuré pour identifier une respiration perturbée par le sommeil (SDB) en utilisant la série chronologique de données.
